# EUROPEAN PATENT APPLICATION

(11) **EP 1 785 151 A1**
(43) Date of publication of application: **16.05.2007**
(21) Application number: 06255740.0
(22) Date of filing: 08.11.2006
(51) Int. Cl.: A61L 2/26, A61L 9/04, C02F 1/44

(54) **Heat sanitization for reverse osmosis systems**

(30) Priority: 09.11.2005 US 270740
(71) Applicant: Mar Cor Purification, Inc., Skippack, PA 19474 (US)
(72) Inventor: Weatherill, David, Burlington, Ontario L7N 3P6 (CA)
(74) Representative: Hedley, Nicholas James Matthew

(57) **Abstract**

A heat sanitization system for a reverse osmosis system (46,48) that utilizes the pure water produced by a reverse osmosis unit to sanitize the various conduits and components of the reverse osmosis system. Pure water from the reverse osmosis unit is collected in a storage tank (86) during a tank fill cycle and, during one of the sanitization cycles, that pure water is withdrawn from the storage tank, heated to a desired sanitizing temperature and the heated, pure water is circulated through a selected flow path of the reverse osmosis system for a sufficient period of time to carry out sanitization. A control system (82) allows the user to select the particular flow path for the sanitization cycle or the flow path can be automatically determined by the control system. There are also cool down cycles that selectively cool the particular flow path after a sanitization cycle.

## Description

### Background

The present invention relates to a system for sanitizing reverse osmosis systems and, more particularly, to a system for utilizing the pure water produced by the reverse osmosis system to sanitize that system by heating that pure water to an elevated temperature and circulating that heated, pure water selectively through flow paths of the reverse osmosis system.

Reverse osmosis systems, in general, produce pure water that is used in dialysis machines, normally with an additive such as sodium bicarbonate. The pure water is circulated through various apparatus, components and conduits and the like in order to provide that pure water to individual dialysis machines. A problem therefore arises in that the apparatus, components and conduits can become contaminated with harmful matter, that is, there can be present bacteria, glucans and the like can affect the pure water circulated therein and downgrade the purity of that water. The bacteria fragments can trigger T-helper cells to create C-reactive proteins that are suspected to lead to cardio-vascular disease, arthritis and carpal tunnel syndrome, among other ailments. It is, therefore, necessary to periodically clean the flow paths through the various apparatuses, components and conduits to eliminate the problem so as to assure that only pure water is produced and circulated through the dialysis machines.

One current method of carrying out that cleaning process is to utilize chemical agents that are circulated through the various flow paths of the dialysis system, however, there is also a downside with such method in that it then becomes necessary after the cleaning with chemical agents has been completed, to then rid the flow paths of the chemical agents themselves, since those chemical agents can be a source of contamination and harmful to the supply of the pure water. As such, the use of chemical disinfectants is a procedure that is costly, time consuming and difficult. Accordingly, while the use of chemical agents alleviate the contaminant problem, the chemical agents thereupon become another source of contamination that must be thoroughly removed before the reverse osmosis system can again supply pure water to the dialysis machines.

The water being produced by the reverse osmosis system, as stated, is pure and therefore is a good medium to carry out the sanitizing function and, as a standard, such water is capable of producing sufficient cleaning if utilized at a temperature of 80 degrees C for a period of at least about ½ hour. Some systems have suggested the use of heated water in a pasteurization system to clean components and conduits of a reverse osmosis system, see U.S. Patent 6,251,279 of Peterson et al, however, in such system, only a portion of the system is disinfected and there is no versatility or selection on the part of the user to clean one or more selected flow paths in the system.

Accordingly, it would be advantageous to have a system that uses the pure water normally produced by the reverse osmosis system to sanitize the system, either in its entirety or by means of certain selected portions and components of the reverse osmosis system.

### Summary of the Invention

The present invention relates to a sanitizing system for use with a reverse osmosis system where the water that is purified by the reverse osmosis system is collected in a storage tank. During a sanitizing cycle, the pure water held with the storage is removed, heated to the sanitization temperature and then circulated through one or more selected flow paths in the reverse osmosis system including a path that passes through the reverse osmosis membrane unit, the delivery conduit having connections to supply water to the dialysis machines and the like. The reverse osmosis system as well as the sanitizing system is constructed of materials that are compatible with the high temperatures and pressures used in the sanitizing cycles of the present invention.

By the present system, the user can select one or more individual flow paths or there is a control system that can carry out an automatic selection of the flow path or flow paths for the sanitization process by opening and closing the appropriate valves and components to pass the heated, pure water though the selected flow path or flow paths. The control system can also be utilized to operate the sanitizing cycles automatically, such as by a timer that initiates one or more of the sanitizing cycles at a predetermined time of day i.e. when the dialysis machines are likely to be inactive or a predetermined time interval i.e. once a week. The control system can then initiate the sanitizing cycle in any order determined by the user and carried out automatically.

In an exemplary embodiment, the heating of the pure water removed from storage tank is carried out at a predetermined maximum rate so that a rapid change of temperature does not create a thermal shock to the system. As such, the heater can heat the pure water at the rate of between about 1-5 degrees C per minute so that the heating of the water used in the sanitizing cycle is gradually heated up to the sanitizing temperature at a relatively slow, controlled rate. In addition the temperature is controlled to assure that there is a maximum allowed temperature during the sanitizing cycles, preferably about 99 degrees C such that the particular sanitizing cycle is discontinued prior to adding enough energy to overcome the latent heat of vaporization.

With each of the sanitizing cycles for the various flow paths, there is also a cool down cycle, again which may be selective as to a desired flow path or flow paths, that circulates the cooler water through the flow paths to flush and cool the flow paths and which allows the heated water from any of the sanitizing cycles to be discharged into a municipal or other system at a temperature that will be acceptable to that system.

These and other features and advantages of the present invention will become more readily apparent during the following detailed description taken in conjunction with the drawings herein.

### Brief Description of the Drawings

FIG. 1 is a schematic view of a reverse osmosis system illustrating the various components used in that system during the normal function of the system in producing pure water for use in dialysis machines;
FIG. 2 is a schematic view of a reverse osmosis system illustrating the heat sanitization tank fill cycle used in the operation of the sanitizing system of the present invention;
FIG. 3 is a schematic view of a reverse osmosis system illustrating the loop and dialysis machines heat sanitization cycle used in the operation of the sanitizing system of the present invention;
FIG. 4 is a schematic view of a reverse osmosis system illustrating the reverse osmosis and loop sanitization cycle used in the operation of the sanitizing system of the present invention;
FIG. 5 is a schematic view of a reverse osmosis system illustrating the reverse osmosis heat sanitization cycle used in the operation of the sanitizing system of the present invention;
FIG. 6 is a schematic view of a reverse osmosis system illustrating the loop cool down cycle used in the operation of the sanitizing system of the present invention;

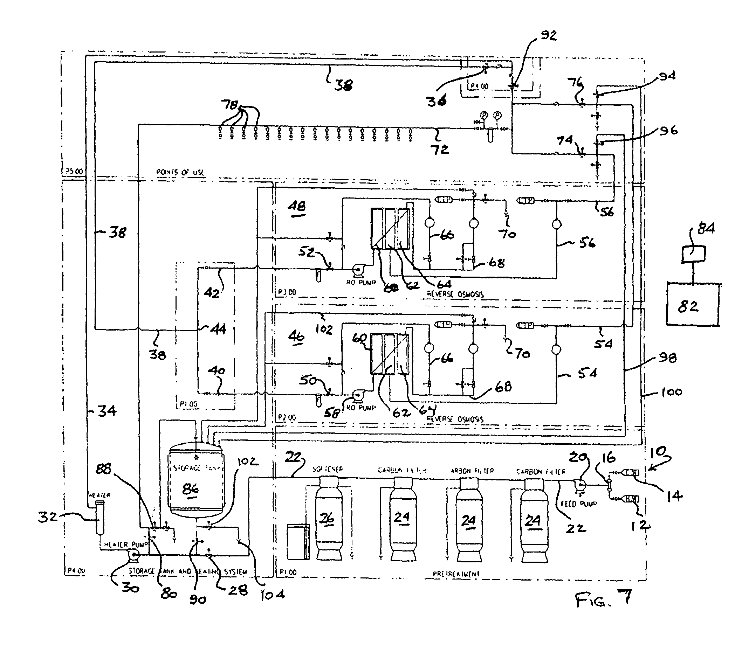

FIG. 7 is a schematic view of a reverse osmosis system illustrating the reverse osmosis and loop cool down cycle used in the operation of the sanitizing system of the present invention; and
FIG. 8 is a schematic view of a reverse osmosis system illustrating the reverse osmosis cool down cycle used in the operation of the sanitizing system of the present invention

### Detailed Description of the Invention

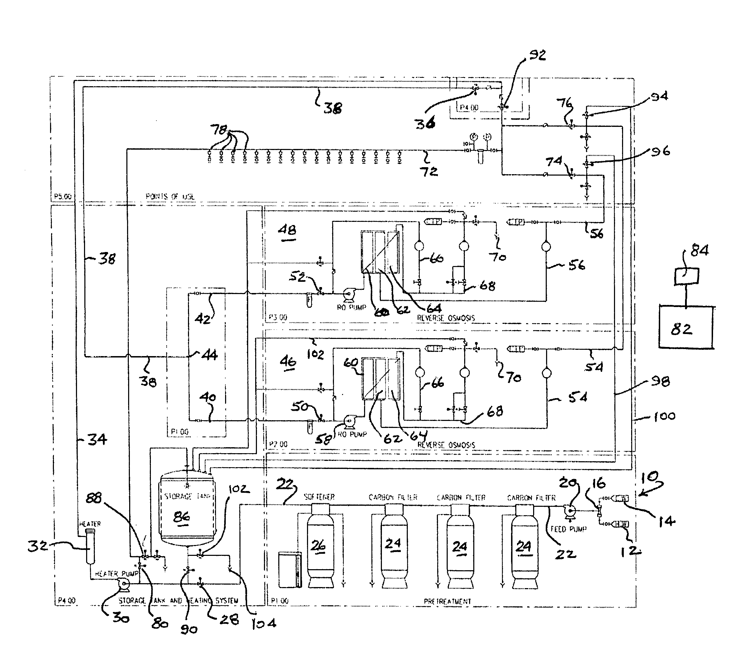

Referring now to FIG. 1, there is shown a schematic view illustrating the normal operation of a reverse osmosis system having the sanitizing system of the present invention incorporated there into. As can be seen, there is an inlet 10 for water and, in the embodiment shown, the inlet comprises a hot water inlet 12 and a cold water inlet 14 and a mixing valve 16 that receives the water from the hot and cold inlets 12, 14 to arrive at a flow of water at a desired temperature, such as 25 degrees C that enters conduit 18. A feed pump 20 raises the pressure of the water to about 45 psi and the water is thereafter passed, by means of conduit 22 through a pretreatment section that preferably includes a plurality of carbon filters 24 and a water softener 26.

In the exemplary embodiment, three carbon filters 24 are shown and which allows the normal system to bypass one of the carbon filters so that it will remain dry and available for use in the event of a failure of one of the carbon filters in use or in the event one of the carbon filters 24 is simply scheduled to be out of service for normal maintenance.

The thus treated water then passes through an open valve 28, heater pump 30 and a heater 32 and enters conduit 34 to pass through an open valve 34 where the water, in conduit 38 splits into inlet conduits 40, 42 at a tee 44. As also can be seen there are pair of reverse osmosis units 46 and 48 that are connected, respectively to inlet conduits 40 and 42 through valves 50 and 52. The reverse osmosis units 46, 48 are constructed with the same components and both have inlet conduits 40 and 42, and outlet conduits 54, 56. Since the components are the same, only one of the reverse osmosis membrane units will be explained and like numbers will be used for the corresponding components.

Taking, therefore, the reverse osmosis unit 46, the water enters via the inlet conduit 38, valve 50 and passes through a high pressure pump 58 where the pressure of the water is raised to about 235 psi. The pressurized water is then passed through a series of membrane vessels 60, 62, 64 where the reverse osmosis takes place to purify the water. The materials of the reverse osmosis units as well as other components that are subjected to the elevated temperatures of water used in carrying out the present invention, such as the membranes, membrane housings, pumps, conduits, fittings, valves, instruments and the like are designed to withstand that temperature. For example, the membranes used in the reverse osmosis unite are cast in polyamide material and constructed in a thin film composition configuration and also, to withstand the elevated temperatures, the membranes are manufactured with special adhesives, permeate tubes and connectors.

As is known, while three membrane vessels are disclosed in the exemplary embodiment, there may be a greater or lesser number of membrane vessels depending, generally, on the number of dialysis machines that are being supplied with the pure water. Normally, the number of membrane vessels may range from one to about fourteen vessels. The is also, normally a feedback loop 66 in order to maintain a constant flow of water through the membrane vessels 60, 62, 64. There is also a drain conduit 68 terminating in a drain 70 for the membrane vessels 60, 62, 64.

Accordingly, the water is purified as it pass through the membrane vessels 60, 62, 64 and the pure water is passed to the outlet conduits 54, 56 where the pure water enters a dialysis conduit 72 through valves 74, 76, where there are connections 78 for providing fluid connections to individual dialysis machines (not shown). The valves 74, 76 enable the system to direct the water from either or both of the reverse osmosis units 46, 48 to the dialysis conduit 72. By such means, either of the reverse osmosis units 46, 48 can be on stream while the other can be off stream for maintenance, or the like, so that there is no downtime in the use of the reverse osmosis system during maintenance. Continuing, the water is returned via the dialysis conduit 72 through open valve 80 to the conduit 22 for recirculation.

As has now been explained, the reverse osmosis system provides the pure water, as described, in order to supply the pure water necessary for the functioning of the dialysis machines.

The sanitizing system therefore can be described and which is utilized to selectively pass heated, pure water through one of more of the previously described paths of water through the reverse osmosis system. The various selective cycles are hereafter described individually. In the operation of al of the following cycles, as well as the overall control of the reverse osmosis system itself, there is a control system 82 having a central processing unit (CPU) that is used to control the various valves, proved a control of power to the components, including pumps and the like and which can be controlled by an input 84 from a user to carry out the settings of valves for all of the cycles to be explained. Thus, the valves hereinafter discussed are controllable by means of an electrical signal that is, in turn, controlled by the control system 82 as well as the operations of the other electrically controlled components.

### Heat Sanitization Tank Fill Cycle

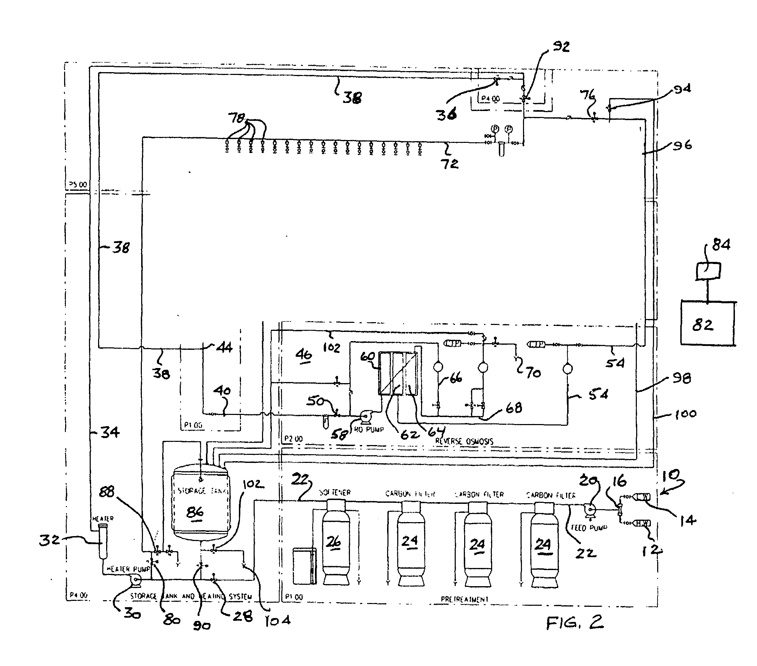

Turning now to Fig. 2, taken along with Fig. 1, there is shown a schematic view of the various components illustrated in Fig. 1 that are employed during the heat sanitization tank fill cycle. As can be seen, the use of the pretreatment is similar to the normal functions as described in Fig. 1, and the same reference numbers used, that is, the water enters the system in the normal manner and passes through the carbon filters 24 and the water softener 26. The water continues by passing through the conduit 22, through the heater pump 30 and the heater 32, through the conduit 34, through open valve 36 and through the conduit 38 where the water enters into one of the reverse osmosis units 46 or 48. Taking the reverse osmosis unit 46 as an example, the water then progresses through the outlet conduit 54, the open valve 76, and the dialysis conduit 72. The water thereafter flows to a storage tank 86 through open valve 88, it begin noted that valve 80 has now been closed. As such the pure water, having passed through reverse osmosis unit 46 fills the storage tank 86.

The storage tank 86 may, during the normal operation of the reverse osmosis system, be empty so as to conserve water as well as avoid a likely location for the growth of bacteria. However, alternatively, there may be instances where the user wants to collect pure water in the storage tank 86 for other purposes and, as such, the storage tank 86 may initially contain a quantity of pure water. Accordingly, by the use of the heat sanitization tank fill cycle, the storage tank 86 is filled with pure water and the level of the water can be monitored by a sensor, not shown, such that the filling of the storage tank 86 can be automatically terminated when the desired quantity or level of water is present in the storage tank 86. It should be noted that, during the heat sanitization tank fill cycle, the heater 32 is not energized and the heater pump 30 is also not in operation. In addition, the dialysis machines are disconnected from the connections 78 or otherwise not in fluid connection with the dialysis conduit 72 so that the water does not enter into any of the dialysis machines.

### Loop and Dialysis Machines Heat Sanitization Cycle

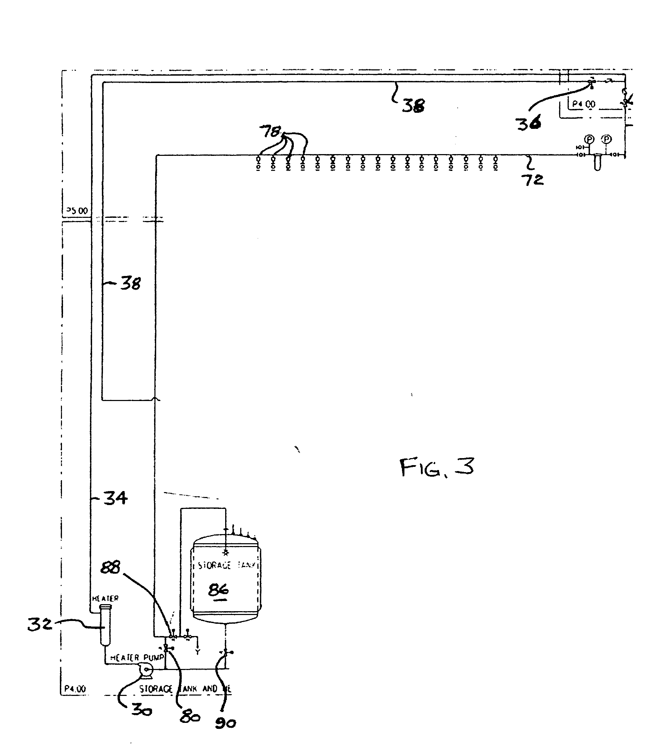

Turning now to Fig. 3, there is shown a schematic view of a loop and dialysis machine heat sanitization cycle of the present invention. With the storage tank 86 filled to the desired capacity with pure water, the operation of the sanitization cycles of the present invention can be explained. As can be seen, the valve 28 is closed, thereby shutting off the supply of water from the inlet 10. Valve 90 is opened so that the water can exit the storage tank 86 to be used in the sanitization process. That pure water from the storage tank 86 is pressurized by the heater pump 30 that has been activated and the pressurized water passes through the heater 32 where it is heated to the desired sanitization temperature.

As shown, the heater 32 is a separate component, however, the heater 32 can be incorporated into or located within the storage tank 86 so as to heat the water contained within the tank 86 to the sanitization temperature and then routed to the heater pump 30. The heater 32 itself can be any one of a variety of heaters, such as an electric heater, a steam heater or the like and is, as discussed, controlled by the control system 82. Accordingly, in the control of the heater 32, the control system 82 can continuously monitor the temperature of the water leaving the heater and control the temperature to the desired level. In addition, the control system 82 can cause heater 32 to heat the water at a predetermined maximum rate, that is, in order to prevent a rapid change in the water temperatures through the various flow paths in the reverse osmosis system, thereby creating a thermal shock, the heater 32 is controlled so that the temperature of the water is raised at the maximum rate of 5 degrees C per minute. The control of the ultimate desired temperature, that of about 80 to 85 degrees C can be controlled by having a temperature sensor at or proximate to the inlet and outlet of the heater 32 so that the control system 82 can sense both temperatures and adjust the heater 32 accordingly to provide the desire heating rate as well as the ultimate temperature of the heated water.

Continuing the cycle, the hot, pure water is carried by conduit 34 to valve 36 which is now closed and valve 92 opened such that the water can directly enter the dialysis conduit 72 where it continues through that conduit and returns to the storage tank 86 through the open valve 88. As such, the heated, pure water has, during that cycle passed through the dialysis conduit 72 to sanitize that flow path through the reverse osmosis system. As indicated, the flow of the heated, pure water is controlled by the control system 82 for a sufficient period of time that the flow path is sanitized by the pure heated water. During this cycle it is also possible to pass the heated, pure water through one or more of the dialysis machines that are fluidly connected to the dialysis conduit 72 through one or more of the connections 78 so that the dialysis machines themselves can be sanitized and the control system 82 can provide a signal to the personnel attending to the dialysis machines to alert that personnel that the dialysis machines can be attached to the connections 78 during this cycle.

As described, this cycle circulates the heated, pure water through a selected portion of the flow path or flow paths through the overall reverse osmosis system and the particular flow path can be selected by the user and entered by means of the input 84 to the control system 82 that then opens and closes the various valves to carry out the selective cycle for sanitization of the particular flow path. Thus, as will be seen with the input 84 to control system 82, the user can select one of the flow paths individually or all of the flow paths simultaneously for circulation of the heated, pure water by means of the control system 82 that operated the particular valves and controls the heating of the pure water in accordance with the user's input. Alternatively, of course any one or more of the sanitization cycles can be commenced by a timer such that the use merely initiates a sanitization cycle and the system carries out one or more cycles based on a timer.

As a still further alternative, even the initiation of the sanitization cycle can be by a timer such that the system undergoes a time sanitization cycle, for example, once a week, without the intervention of the user and such cycling would normally be initiated by the timer at times when the dialysis machines are not normally in use.

### Reverse Osmosis and Loop Heat Sanitization Cycle

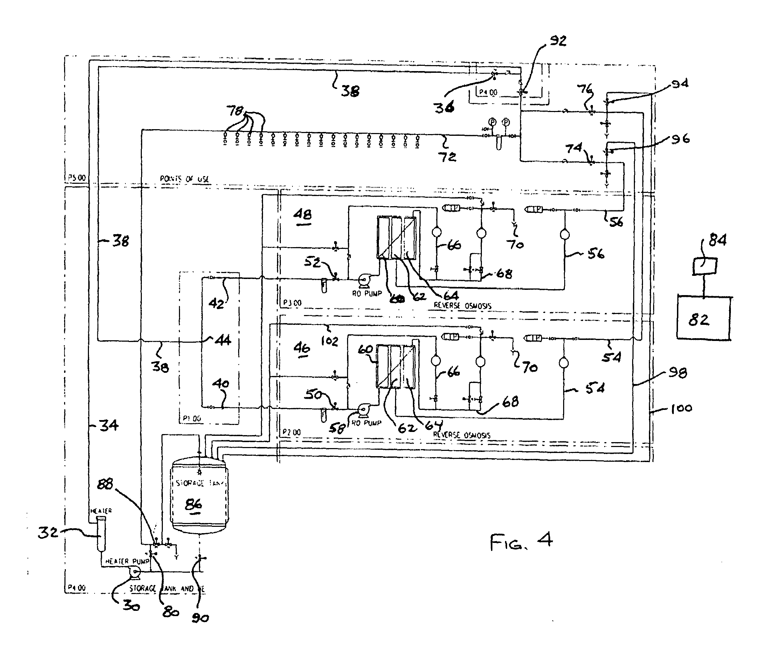

Turning now to Fig. 4, there is shown a schematic view of the reverse osmosis and loop heat sanitization cycle of the present invention. When this cycle is selected, the reverse osmosis units and the loop having the connections 78 to the dialysis machines are heat sanitized. Again, as with the prior sanitization cycle, the valve 28 is closed, thereby shutting off the supply of water from the inlet 10. Valve 90 is opened so that the water can exit the storage tank 86 to be used in the sanitization process. That pure water from the storage tank 86 is pressurized by the heater pump 30 that has been activated and the pressurized water passes through the heater 32 where it is heated to the desired sanitization temperature and at the desired rate.

Continuing the cycle, the hot, pure water is carried by conduit 34 through the open to valve 36, valve 92 being closed, such that the water passes along conduit 38 to tee 44 where the water can then pass though both of the inlet conduits 40, and 42 since both valves 50 and 52 are open so that the heated, pure water can enter and continue through both of the reverse osmosis units 46, 48 to heat sanitize those units and the water can continue through the outlet conduits 54, 56, through now opened valves 74 and 76 to enter the dialysis conduit 72 where it continues through that conduit and returns to the storage tank 86 through the open valve 88. During this cycle, the high pressure pump 58 is not energized since there is no need for the high pressure for the water to pass through the membrane vessels 60, 62, 64.

As such, the heated, pure water has, during that cycle, passed through the dialysis conduit 72 as well as both of the reverse osmosis units 46, 48 to sanitize that flow path through the reverse osmosis system. Again, the flow of the heated, pure water is controlled by the control system 82 for a sufficient period of time that the flow path is sanitized by the pure heated water. During this cycle, as with the prior cycle, it is still possible to pass the heated, pure water through one or more of the dialysis machines to sanitize those dialysis machines in the manner described and with the signal to assert the personnel that the dialysis machines can be attached to the connections 78 during this cycle.

### Reverse Osmosis Heat Sanitization Cycle

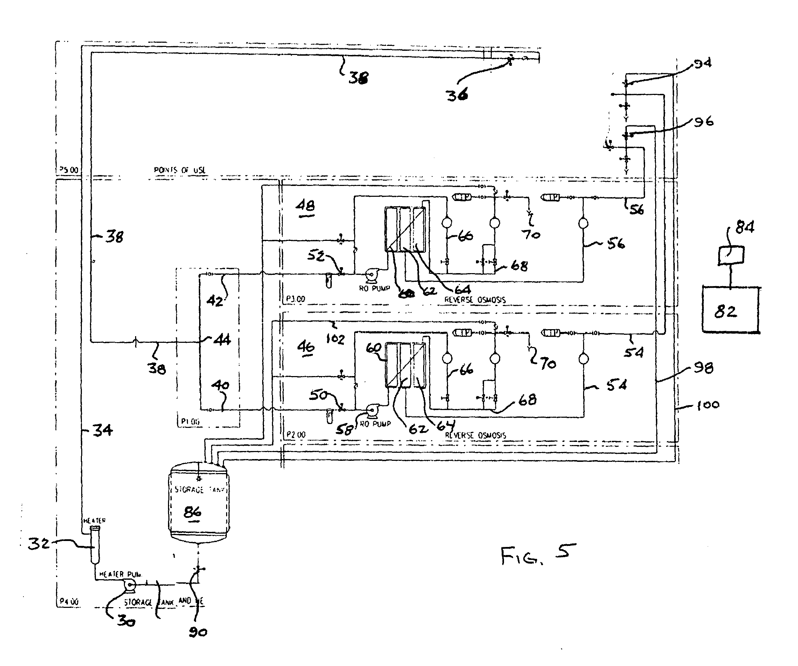

Turning now to Fig. 5, there is shown a reverse osmosis heat sanitization cycle of the present invention. This further cycle can be carried out, whether initiated manually or automatically, by passing the heated, pure water along a flow path that is only through the reverse osmosis units 46, 48. Again the cycle is carried out by closing the valve 28, thereby shutting off the supply of water from the inlet 10. Valve 90 is opened so that the water can exit the storage tank 86 and, again, that water is pressurized by the heater pump 30 and the pressurized water passes through the heater 32 where it is heated to the desired sanitization temperature and at the maximum rate.

Continuing the cycle, the heated, pure water is carried by conduit 34 through open valve 36, valve 92 being closed, such that the water passes along conduit 38 to tee 44 where the water can then pass though both of the inlet conduits 40 and 42 since both valves 50 and 52 are open so that the heated, pure water can enter and continue through both of the reverse osmosis units 46, 48 to heat sanitize those units and the water can continue through the outlet conduits 54, 56. With this cycle, however, the valves 74 and 76 are closed and valves 94 and 96 are opened so that the water can continue through the conduits 98, 100 and return to the storage tank 86. As a further path of the water during this cycle, the water that is normally may be drained through drain 70 continues to circulate in conduit 102 to the storage tank 86 for both of the reverse osmosis units 46, 48.

Accordingly, as can now be seen, the control system 82 can take the reverse osmosis sanitizing system through various cycles in carrying out the sanitization of differing flow paths in the reverse osmosis system and thus, the user has the flexibility to select a particular flow path, whether in sanitizing the reverse osmosis units, the loop wherein the dialysis machines are fluidly attached or the like. The cycles can each be selected by the user or can be triggered automatically by the control system 82. In each case, the pure water, that has passed through a reverse osmosis unit and has been accumulated in the storage tank 86, is, during the particular sanitization cycle, pressurized and pumped through the particular selected flow path in the reverse osmosis system.

After each of the above described sanitization cycles, there is a "cool down" cycle that allows the reverse osmosis system to cool down to its normal operating temperatures and the cool down cycles use the lower temperature water that is supplied via the inlet of the reverse osmosis system and which is at the operating temperature of the system. The "cool down" cycles will, therefore be hereafter described.

### Loop Cool Down Cycle

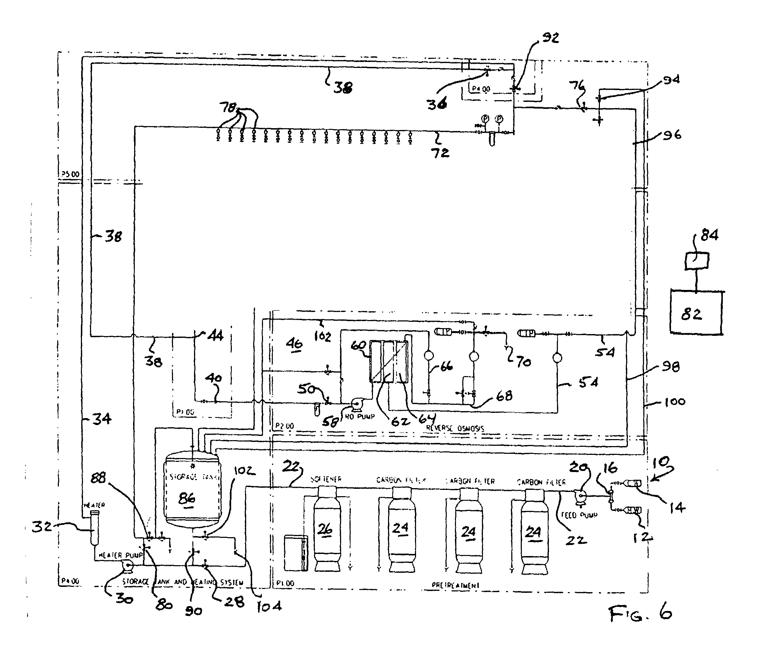

Turning, therefore, to Fig. 6, there is shown a schematic view of the loop cool down cycle that is used with the present invention. With this cycle, valve 28 is now opened and valve 90 closed thereby shutting off the water from the storage tank 86 and opening the system to the flow of water from the inlet 10 and which passes through the water pretreatment portion of the reverse osmosis system, that is, the water passes through at least one, preferably two, of the carbon filters 24 and the water softener 26. That cooler water, normally at about 00 degrees C, then passes through the heater pump 30 that has been inactivated and through the heater 32 which has also been inactivated.

Continuing the cycle, the water is carried by conduit 34 through open valve 36, valve 92 being closed, such that the water can continue along conduit 38 to tee 44 and into the inlet conduit 40 to enter and pass through the reverse osmosis unit 46. As noted, at tee 44, the water can pass through either or both of the inlet conduits 40, 42 depending on the position of the valves 50, 52. Thus, depending on whether only one or both of the reverse osmosis units 4, 46 are to be cooled down, the user can select one or both of the valves 50, 52.

Taking, again, the description of the reverse osmosis unit 44, cooler water can pass through the membrane vessels 60, 62, 64 and deliver the water to the outlet conduit 54. Thereafter, since valve 76 is open and valve 94 is closed, the cooler water can pass through the dialysis conduit 72, past the connections 78 and continue through the open valve 88 (valve 80 is closed) to enter the storage tank 86. The cooler water thereby cools any remaining heated water remaining in the storage tank 86 after a sanitization cycle so that the water in the storage tank 86 can be reduced to a temperature where it can be discharged safely through the open valve 102 via a drain 104. As an example, normally at the initiation of the cool down cycle, the storage tank 86 may be three quarters filled with water at a temperature of about 65 degrees C and the cooler water is introduced into the storage tank 86 at about 25 degrees C such that the cooler water reduces the temperature of the water in the storage tank 86 to allow that water to be safely drained into a commercial municipal drainage system.

Again the flow of the cooler pure water is controlled by the control system 82 for a sufficient period of time that the various flow paths are cooled by the cooler pure water.

As described, this cycle circulates the cooler water through a selected portion of the flow path or flow paths of the overall reverse osmosis system and, as with the sanitization cycles, the particular flow path can be selected by the user and entered by means of the input 84 to the control system 82 to control the opening and closing of the various valves to carry out the selective cycle for cooling of the particular conduits and components in the selected flow path. Thus, as will be seen with the input 84 to control system 82, the user can select one of the flow paths individually or all of the flow paths simultaneously for circulation of the cooler water by means of the control system 82 that operated the particular valves and controls the flow paths of the water in accordance with the user's input. Accordingly, as now described, this cycle circulates the cooler water from the inlet 10 through at least one of the reverse osmosis units and also through the loop where the dialysis machines are connected.

### Reverse Osmosis and Loop Cool Down Cycle

Turning now to Fig, 7 there is shown a schematic view of the reverse osmosis and loop cool down cycle. With this cool down cycle, the reverse osmosis units and the loop having the connections 78 to the dialysis machines are cooled down. Again, as with the prior cool down cycle, valve 28 is opened and the valve 90 closed thereby allowing the cooler water to enter the reverse osmosis system from the inlet 10.

Continuing the cycle, the cooler water is carried by conduit 34, through open valve 36, valve 92 being closed, such that the water passes through conduit 38 to tee 44 where the water can then pass though both of the inlet conduits 40 and 42 since both valves 50 and 52 are open so that the cooler water can enter and continue through both of the reverse osmosis units 46, 48 to cool those units. The cooler water can continue through the outlet conduits 54, 56, through now opened valves 74 and 76 to enter the dialysis conduit 72 where the cooler water continues through that conduit and returns to the storage tank 86 through the open valve 88, valve being 80 closed.

As such, the cooler water has, during that cycle, passed through the dialysis conduit 72 as well as both of the reverse osmosis units 46, 48 to cool the various conduits and components of the reverse osmosis system. Again, the flow of the cooler water is controlled by the control system 82 for a sufficient period of time that the flow path is cooled by the pure heated water and the admission of the cooler water into the storage tank 86 serves to cool the water in the storage tank 86 down to a temperature where it can be safely discharged via drain 104 into a municipal waste system.

### Reverse Osmosis Cool Down Cycle

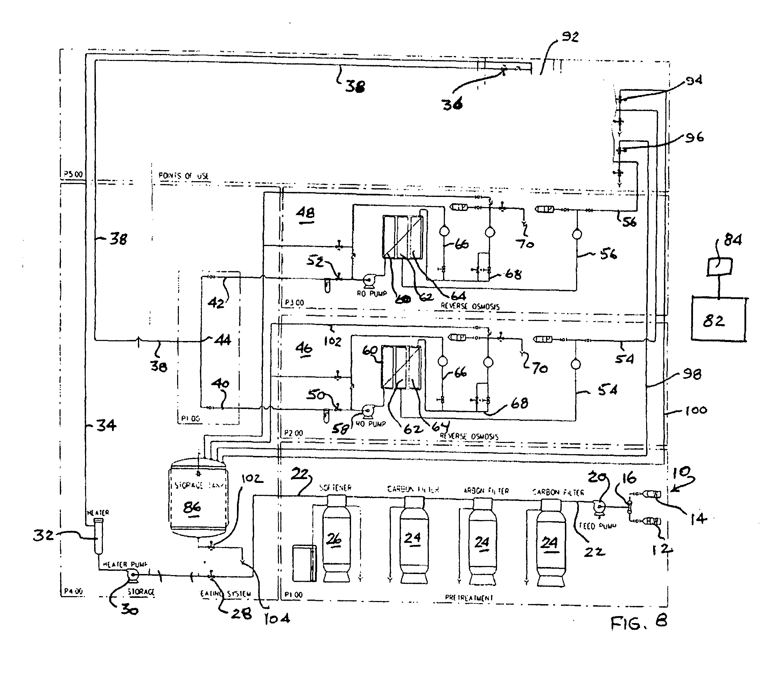

Turning finally to Fig. 8, there is show a schematic view of a reverse osmosis cool down cycle in accordance with the present invention. Again, this cycle is carried out by opening the valve 28 and closing the valve 90 thereby admitting the cooler water to the inlet 10 to the reverse osmosis system. The cooler water thereafter passes through inactive heater pump 30 and inactive heater 32 to conduit 34 and then through open valve 36, valve 92 being closed, such that the water passes along conduit 38 to tee 44 where the water can then pass though both of the inlet conduits 40 and 42 since both valves 50 and 52 are open so that the cooler water can enter and continue through both of the reverse osmosis units 46, 48 to cool the conduits and components within those units and the water can continue through the outlet conduits 54, 56. With this cycle, however, the valves 74, 76 are closed and valves 94, 96 are opened so that the water can continue through the conduits 98, 100 and return to the storage tank 86. As a further path of the water during this cycle, the water that normally may be drained through drain 70 continues to circulate in conduit 102 to the storage tank 86 for both of the reverse osmosis units 46 and 48.

Accordingly, as can now be seen, the control system 82 can take the reverse osmosis sanitizing system through various cool down cycles in carrying out the cool down of differing flow paths in the reverse osmosis system subsequent to a sanitization cycle and thus, the user has the flexibility to select a particular flow path, whether in cooling the reverse osmosis units, the loop wherein the dialysis machines are fluidly attached or the like. The cool down cycles can each be selected by the user or can be triggered automatically by the control system 82. In each cycle, however, by the use of the cooler water, the ultimate water that is discharged into a municipal sanitation system is at an acceptable temperature for such discharge.

Those skilled in the art will readily recognize numerous adaptations and modifications which can be made to the sanitization system for a reverse osmosis system of the present invention which will result in an improved sanitization thereof, yet all of which will fall within the scope and spirit of the present invention as defined in the following claims. Accordingly, the invention is to be limited only by the following claims and their equivalents.

## Claims

1. A sanitizing system for use with a reverse osmosis system having an inlet for receiving water, at least one reverse osmosis membrane unit adapted to receive water from said inlet, purify the water and deliver the water to a delivery conduit and at least one means for making a fluid connection to a dialysis machine, the sanitizing system comprising a storage tank for receiving and storing pure water from the at least one reverse osmosis membrane unit, a heater and a control system wherein the control system activates a sanitizing cycle wherein pure water is withdrawn from the tank, heated by the heater and passed through the reverse osmosis system.

2. The sanitizing system of claim 1 wherein the reverse osmosis system has a plurality of flow paths for water and said control system can selectively pass the heated water from the tank through one or more of the flow paths.

3. The sanitizing system of claim 2 wherein said control system can pass water that has not been heated by the heater selectively through one of the plurality of flow paths.

4. The sanitizing system of claim 1 wherein the reverse osmosis system has a flow path that passes the heated water from the tank through the at least one reverse osmosis membrane unit and said control system activates the sanitizing cycle to pass the pure water through the at least one reverse osmosis membrane unit.

5. The sanitizing system of claim 1 wherein the reverse osmosis system has a flow path that passes the heated water from the tank through the delivery conduit and said control system activates the sanitizing cycle to pass the pure water through the delivery conduit having at least one means for making a fluid connection to a dialysis machine.

6. The sanitizing system of claim 1 wherein the control system is controlled by the user to pass the heated water from the tank through either the at least one reverse osmosis membrane unit or the delivery conduit having at least one means for making a fluid connection to a dialysis machine

7. The sanitizing system of claim 1 wherein the heater is controlled so as to control the rate of temperature rise of the water passing therethrough.

8. A method of sanitizing a reverse osmosis system, said method comprising the steps of:
providing a reverse osmosis system having an inlet for receiving water at a controlled temperature, a delivery conduit having at least one means for making a fluid connection to a dialysis machine and at least one reverse osmosis membrane unit wherein water can be passed from the inlet to the delivery conduit through the at least one reverse osmosis membrane unit to deliver pure water to the delivery conduit;
directing a flow of pure water from the reverse osmosis membrane unit into a tank; and
withdrawing and heating a flow of water from the tank and directing that heated water through the reverse osmosis system to sanitize at least a portion of the reverse osmosis system.

9. The method as defined in claim 8 wherein the step of withdrawing and heating a flow of water comprises providing a heater downstream of the tank to heat the water withdrawn from the tank to at least a predetermined temperature.

10. The method as defined in claim 8 wherein the step of withdrawing and heating a flow of water comprises heating the water at a controlled rate so as to not exceed a predetermined rate of temperature rise.
